Europäisches Patentamt
European Patent Office
Office européen des brevets

(19)

(11) Publication number: **0 323 869 A1**

(12) # EUROPEAN PATENT APPLICATION

(21) Application number: **89200009.2**

(22) Date of filing: **02.01.89**

(51) Int. Cl.4: **C 07 D 275/06**
C 07 D 417/06, A 01 N 43/80

(30) Priority: **06.01.88 GB 8800223**
**06.01.88 GB 8800224**
**06.01.88 GB 8800225**

(43) Date of publication of application:
**12.07.89 Bulletin 89/28**

(84) Designated Contracting States:
**AT BE CH DE ES FR GB GR IT LI LU NL SE**

(71) Applicant: **SHELL INTERNATIONALE RESEARCH MAATSCHAPPIJ B.V.**
**Carel van Bylandtlaan 30**
**NL-2596 HR Den Haag (NL)**

(72) Inventor: **Haddock, Ernest**
**187 Queensborough Road Sheerness**
**Isle of Sheppey Kent ME12 3EL (GB)**

(74) Representative: **Hunter, Keith Roger Ian et al**
**4 York Road**
**London SE1 7NA (GB)**

(54) **Saccharin derivatives.**

(57) The invention provides saccharin derivatives of the general formula

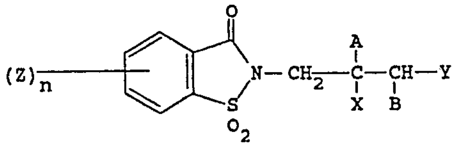

(I)

in which A and B independently represent a hydrogen or halogen atom or together represent a single carbon-carbon bond; X represents a hydrogen or halogen atom or an optionally substituted alkyl group or together with A represents an alkenyl group or together with B represents a -O- group; Y represents a hydrogen or halogen atom, a nitro, cyano, hydroxyl, formyl or carboxyl group or an optionally substituted alkyl, alkenyl, alkynyl, alkoxy, amino, alkylamino, dialkylamino, alkoxycarbonyl, alkanoyl, aroyl, alkylthio, alkylsulphinyl, alkylsulphonyl, carbamoyl, alkylamido, aryl, aralkyl or heterocyclyl group; each Z represents a halogen atom, nitro, cyano, hydroxyl, alkyl, haloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, carbamoyl or alkylamido group; and n represents an integer from 0 to 4; with the proviso that A, B and X or A, X and Y do not simultaneously represent a hydrogen atom; processes for their preparation; compositions containing such compounds and their use as fungicides.

EP 0 323 869 A1

**Description**

## SACCHARIN DERIVATIVES

This invention relates to certain saccharin derivatives, a process for their preparation, compositions containing such compounds and their use as fungicides.

According to the present invention there is provided a compound of the general formula

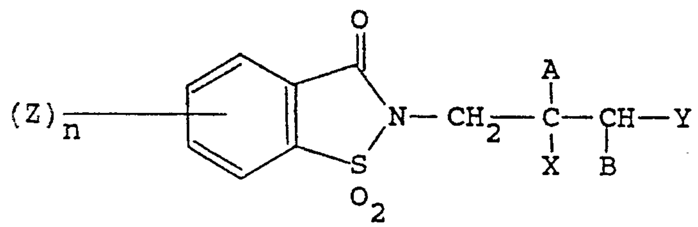

(I)

in which A and B independently represent a hydrogen or halogen atom or together represent a single carbon-carbon bond; X represents a hydrogen or halogen atom or an optionally substituted alkyl group or together with A represents an alkenyl group or together with B represents a -O- group; Y represents a hydrogen or halogen atom, a nitro, cyano, hydroxyl, formyl or carboxyl group or an optionally substituted alkyl, alkenyl, alkynyl, alkoxy, amino, alkylamino, dialkylamino, alkoxycarbonyl, alkanoyl, aroyl, alkylthio, alkylsulphinyl, alkylsulphonyl, carbamoyl, alkylamido, aryl, aralkyl or heterocyclyl group; each Z represents a halogen atom, nitro, cyano, hydroxyl, alkyl, haloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, carbamoyl or alkylamido group; and n represents an integer from 0 to 4; with the proviso that when A and X are both hydrogen atoms, neither B nor Y represents a hydrogen atom.

These compounds have been found to exhibit fungicidal activity.

When the compounds of this invention contain an alkyl or alkenyl substituent group, this may be linear or branched and may contain up to 12, preferably up to 6, and especially up to 4, carbon atoms.

When any of the foregoing substituents are designated as being optionally substituted, the substituent groups which are optionally present may be any one or more of those customarily employed in the development of pesticidal compounds and/or the modification of such compounds to influence their structure/activity, persistence, penetration or other property. Specific examples of such substituents include, for example, halogen atoms, nitro, cyano, hydroxyl, alkyl, haloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, carbamoyl and alkylamido groups. When any of the foregoing substituents represents or contains an alkyl substituent group, this may be linear or branched and may contain up to 12, preferably up to 6, and especially up to 4, carbon atoms.

It is preferred that X represents a hydrogen or halogen atom or a $C_{1-6}$ haloalkyl, particularly a $C_{1-4}$ haloalkyl, group or together with A represents a $C_{1-4}$ alkenyl group or together with B represents a -O-group.

Preferably, Y represents a hydrogen or halogen atom, a $C_{1-12}$ alkyl, particularly a $C_{1-6}$ alkyl and especially a $C_{1-4}$ alkyl, group, a $C_{1-12}$ alkoxycarbonyl, particularly a $C_{1-6}$ alkoxycarbonyl and especially a $C_{1-4}$ alkoxycarbonyl, group, an aryl, particularly a phenyl group, or a heterocyclylalkyl, particularly a heterocyclyl-$C_{1-6}$ alkyl group and especially a heterocyclyl-$C_{1-4}$ alkyl group, each group being optionally substituted by one or more substituents selected from halogen atoms, nitro, cyano, hydroxyl, $C_{1-4}$ haloalkyl, $C_{1-4}$ alkoxy, amino, $C_{1-4}$ alkylamino, di-$C_{1-4}$ alkylamino, formyl, $C_{1-4}$ alkoxycarbonyl and carboxyl groups.

It is also preferred that n is 0 or 1.

A particularly preferred sub-group of compounds of formula I is that in which A represents a hydrogen, chlorine or bromine atom, B represents a hydrogen, chlorine, bromine or iodine atom or together with A represents a single carbon-carbon bond, X represents a hydrogen, chlorine or bromine atom or a chloromethyl or bromomethyl group or together with A represents a $=CH_2$ group or together with B represents a -O- group; Y represents a hydrogen, chlorine or bromine atom or a methyl, chloromethyl, bromomethyl, tri(chloromethyl)methyl, ethoxycarbonyl, phenyl, triazolylmethyl or 1,1,3-trioxo-benzisothiazol-2-ylmethyl group; and n is O.

Another preferred sub-group of compounds of formula I is that in which A and X independently represent a hydrogen, chlorine or bromine atom; B represents a chlorine or bromine atom or together with A represents a single carbon-carbon bond; Y represents a chloromethyl group; n is 1; and Z represents a fluorine or chlorine atom or a nitro or amino group.

It should also be appreciated that the compounds of formula I are capable of existing as different isomers and that the invention thus includes both the individual isomers and mixtures of such isomers. For instance, compounds of formula I in which A and B together represent a single carbon-carbon bond are capable of

existing as different geometric isomers and certain compounds of formula I are capable of existing as different optical isomers. Indeed, a large proportion of the compounds of formula I contain two asymmetric centres and therefore exist as four optically active isomers which may be separated into two enantiomeric pairs of isomers. Moreover, it has been discovered that the two pairs of enantiomers generally exhibit different levels of fungicidal activity.

A further preferred sub-group of compounds of formula I is therefore that in which A represents a hydrogen or halogen atom; B represents a halogen atom; X represents a hydrogen or halogen atom or an optionally substituted alkyl group; Y represents a halogen atom, a nitro, cyano, hydroxyl, formyl or carboxyl group or an optionally substituted alkyl, alkenyl, alkynyl, alkoxy, amino, alkylamino, dialkylamino, alkoxycarbonyl, alkanoyl, aroyl, alkylthio, alkylsulphinyl, alkylsulphonyl, carbamoyl, alkylamido, aryl, aralkyl or heterocyclyl group; each Z represents a halogen atom, nitro, cyano, hydroxyl, alkyl, haloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, carbamoyl or alkylamido group; and n represents an integer from 0 to 4; with the provisos that A is not the same as X and B is not the same as Y; in the form of isomers having the same stereochemical configuration as that enantiomeric pair of isomers of N-(4-chloro-2,3-dibromobutyl) saccharin which has a melting point of 143°C.

The present invention also provides a process for the preparation of a compound of formula I as defined above which comprises

(a) reacting a compound of the general formula

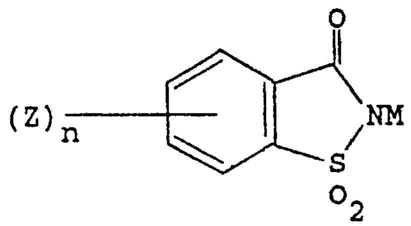

(II)

in which Z and n are as defined above and M represents a hydrogen or metal, particularly sodium or potassium, atom or an $NH_4$ group, with a compound of the general formula

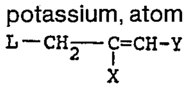

in which X and Y are as defined above and L represents a leaving group, preferably, a halogen atom;

(b) if desired, reacting the compound obtained in (a) with a compound of the general formula

A - B (IV)

in which A and B independently represent a hydrogen or halogen atom; and

(c) if desired, subjecting a compound obtained in (b), in which A, B, X, Y, n and Z are as defined for the further preferred sub group of compounds of formula I referred to above, to isomer separation.

Preferably, the isomer separation is accomplished by

(i) treating a racemic mixture of four isomers of the compound with a solvent which selectively dissolves a first enantiomeric pair of isomers, filtering off a second enantiomeric pair of isomers and then isolating the first enantiomeric pair of isomers; or

(ii) subjecting a racemic mixture of four isomers of the compound to chromatography and isolating the desired isomers.

When M represents a hydrogen atom in the compound of formula II, reaction step (a) is conveniently carried out in the presence of a base. Suitable bases include sodium hydride, sodium hydroxide, potassium hydroxide, potassium carbonate, sodium ethoxide and organic bases such as triethylamine and pyridine.

Step (a) of the process of the invention may be carried out in the presence of a solvent. Suitable solvents include dimethyl formamide, dimethylsulphoxide, sulpholane, N-methylpyrrolidone, pyridine and halogenated hydrocarbons, such as carbon tetrachloride and chloroform. Suitable bases include sodium hydride. This reaction step is suitably carried out at a temperature of 0° C to 150° C, the preferred reaction temperature being 15° C to 120° C.

Step (b) of the process of the invention is also conveniently carried out in the presence of a solvent. Suitable solvents in this case include halogenated hydrocarbons, particularly carbon tetrachloride, methylene chloride and chloroform, aromatic hydrocarbons and alcohols, such as ethanol. This reaction step is suitably carried out at a temperature of 0° C to 80° C, the preferred reaction temperature being 15° C to 30° C.

Compounds of formula II, III and IV are known compounds or can be prepared by processes analogous to known processes.

As previously mentioned, the compounds of general formula I have been found to have fungicidal activity. Accordingly, the invention further provides a fungicidal composition which comprises a carrier and, as active ingredient, a compound of formula I as defined above. A method of making such a composition is also provided which comprises bringing a compound of formula I as defined above into association with at least

one carrier. Such a composition may contain a single compound or isomer or a mixture of several compounds and/or isomers of the present invention.

A composition according to the invention preferably contains from 0.5 to 95% by weight of active ingredient.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating fungicidal compositions may be used.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cylohexanone; ethers; aromatic or araliphatic hydrocarbons, for example benzene, toluene and xylene; petroleum fractions, for example, kerosine and light mineral oils; chlorinated hydrocarbons, for example carbon tetrachloride, perchloroethylene and trichloroethane. Mixtures of different liquids are often suitable.

Fungicidal compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohols or alkyl phenols, for example p-octylphenol or p-octylcresol, with ethylene oxide and/or propylene oxide; sulphates or sulphonates of these condensation products; alkali or alkaline earth metal salts, preferably sodium salts, or sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, of active ingredient and usually contain in addition to solid inert carrier, 3-10% of a dispersing agent and, where necessary, 0-10% w of stabiliser(s) and/or other additives such as penetrants or stickers. Dusts are usually formulated as a dust concentrate having a similar composition to that of a wettable powder but without a dispersant, and may be diluted in the field with further solid carrier to give a composition usually containing $\frac{1}{2}$-10% w of active ingredient. Granules are usually prepared to have a size between 10 and 100 BS mesh (1.676 - 0.152mm), and may be manufactured by agglomeration impregnation techniques. Generally, granules will contain $\frac{1}{2}$-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, where necessary, co-solvent, 1-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise' like consistency.

The composition of the invention may also contain other ingredients, for example, other compounds possessing herbicidal, insecticidal or fungicidal properties.

Of particular interest in enhancing the duration of the protective activity of the compounds of this invention is the use of a carrier which will provide a slow release of the fungicidal compounds into the environment of the plant which is to be protected. Such slow-release formulations could, for example, be inserted in the soil

adjacent to the roots of a plant, or could include an adhesive component enabling them to be applied directly to the stem of a plant.

The invention still further provides the use as a fungicide of a compound of the general formula I as defined above or a composition as defined above, and a method for combating fungus at a locus, which comprises treating the locus, which may be for example plants subject to or subjected to fungal attack, seeds of such plants or the medium in which such plants are growing or are to be grown, with such a compound or composition.

The present invention is of wide applicability in the protection of crop plants against fungal attack. Typical crops which may be protected include vines, grain crops such as wheat and barley, rice, apples and tomatoes. The duration of protection is normally dependent on the individual compound selected, and also a variety of external factors, such as climate, whose impact is normally mitigated by the use of a suitable formulation.

The invention is further illustrated by the following Examples.

## Example 1

Preparation of N-(4-chlorobut-2-enyl)saccharin (A/B = C-C bond; X = H; Y = $CH_2Cl$; n = 0)

Potassium saccharin (4.72g, 0.025 mol) was heated with an excess of cis/trans 1,4-dichlorobut-2-ene at 90° C for 2 days. The mixture was then cooled and poured into water (100ml) and chloroform (100ml). The chloroform layer was separated, dried over magnesium sulphate and the chloroform then evaporated to leave a dark brown oil. This was then chromatographed on silica gel using chloroform as eluant to give cis/trans N-(4-chlorobut-2-enyl) saccharin (2.3g) as a pale yellow solid, m.pt. 86-88° C.

Analysis

| | | | |
|---|---|---|---|
| Calc. | C:48.6; | H:3.7; | N:5.2% |
| Found | C:48.3; | H:3.7; | N:5.2% |

## Example 2

(i) Preparation of N-(4-chloro-2,3-dibromobutyl) saccharin (A=B=Br; X=H; Y=$CH_2Cl$; n=0)

The cis/trans N-(4-chlorobut-2-enyl)saccharin (0.82g, 0.003 mol) obtained in Example 1 was dissolved in carbon tetrachloride (20ml). Excess bromine was then added and the mixture left at room temperature for 16 hours. The carbon tetrachloride solution was then washed with sodium metabisulphite solution (50ml) and water (50ml), separated and dried over magnesium sulphate. The residue was then treated with ethanol (5ml) and N-(4-chloro-2,3-dibromobutyl) saccharin (0.9g) filtered off as white crystals, m.pt. 114-116° C.

Analysis

| | | | |
|---|---|---|---|
| Calc. | C:30.6; | H:2.3; | N:3.2% |
| Found | C:30.7; | H:2.4; | N:3.5% |

(ii) Enantiomeric pair separation Method (1)

Treatment of the N-(4-chloro-2,3-dibromobutyl) saccharin obtained in (i) above with dry ether followed by filtration gave white crystals of the enantiomeric pair of isomers A, m.pt 124° C, which were substantially free of the enantiomeric pair of isomers B.

Evaporation of the solvent gave a pale yellow oil which crystallised on adding hexane to give B as white crystals. Chromatography on silica gel using 1:1 chloroform:hexane as eluant gave pure B, m.pt. 143° C.

Method (2)

The N-(4-chloro-2,3-dibromobutyl)saccharin obtained in (i) above was chromatographed on silica gel using 2:1 methylene chloride:hexane as eluant to give white crystals of B, m.pt. 143° C, in a recovery of 92% and white crystals of A, m.pt. 123° C, in a recovery of 80%.

## Example 3

Preparation of N-(3-bromoprop-2-enyl)saccharin (A/B = C-C bond; X = H; Y = Br; n = 0)

Sodium saccharin (4.1g, 0.02 mol), cis/trans 1,3-dibromoprop-1-ene (4.0g, 0.02 mol) and N,N-dimethylformamide (30 ml) were heated at about 100° C for 16 hours. The mixture was then poured into water (150 ml) and extracted with diethyl ether (2 x 100ml). The ether layer was then washed with water, separated and dried over magnesium sulphate. Evaporation of the ether gave a white solid which crystallised from ethanol as white crystals of cis/trans N-(3-bromoprop-2-enyl) saccharin (3.9 g), m.pt. 61-62° C.

Analysis

| | | | |
|---|---|---|---|
| Calc. | C:39.7; | H:2.6; | N:4.6% |
| Found | C:39.8; | H:2.7; | N:4.8% |

Example 4

Preparation of N-(2,3,3-tribromopropyl)saccharin (A=B=Y=Br; X=H; n=O)

The cis/trans N-(3-bromoprop-2-enyl)saccharin (0.91 g, 0.03 mol) obtained in Example 3, excess bromine and carbon tetrachloride (50 ml) were stirred at 20°C for 16 hours. The mixture was then washed with 10% (w/v) sodium metabisulphite solution (100 ml) and water (100 ml) and the carbon tetrachloride layer separated and dried over magnesium sulphate. Evaporation of the carbon tetrachloride gave a white solid which crystallised from ethanol as white crystals of N-(2,3,3-tribromopropyl)saccharin (1.2g), m.pt 122-123°C.

Analysis

| | | | |
|---|---|---|---|
| Calc. | C:26.0; | H:1.7; | N:3.0% |
| Found | C:26.0; | H:1.9; | N:3.5% |

Examples 5 to 38

By processes similar to those described in Examples 1 to 4 above, further compounds according to the invention were prepared as detailed in Table I below. In this table, the compounds are identified by reference to formula I. Melting point and CHN analysis data for these compounds are given in Table IA.

TABLE I

| Ex. No. | A | X | B | Y | n | Z |
|---|---|---|---|---|---|---|
| 5 | -Br | -H | -Br | -Cl | 0 | - |
| 6 | -Br | -H | -H | -Br | 0 | - |
| 7 | -Cl | -H | -Cl | $-CH_2Cl$ | 0 | - |
| 8 | -Cl | -H | -I | $-CH_2Cl$ | 0 | - |
| 9 | -Br | -H | -I | $-CH_2Cl$ | 0 | - |
| 10 | -H | - O - | | $-CH_2Cl$ | 0 | - |
| 11 | -Br | -H | -Br | $-CH_2Br$ | 0 | - |
| 12 | -Br | $-CH_2Br$ | -H | $-C(CH_2Cl)_3$ | 0 | - |
| 13 | $=CH_2$ | | -H | $-C(CH_2Cl)_3$ | 0 | - |
| 14 | -Br | -H | -Br | $-CH_3$ | 0 | - |
| 15 | -Br | -H | -Br | $-COOC_2H_5$ | 0 | - |
| 16 | -Br | -H | -Br | Phenyl | 0 | - |
| 17 | -Br | -H | -Br | $-CH_2-$ 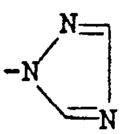 | 0 | - |
| 18 | -Br | -H | -Br | $-CH_2$ 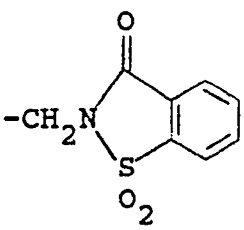 | 0 | - |
| 19 | -Cl | -H | -Cl | $-CH_2Cl$ | 1 | 5-Cl |
| 20 | -Br | -H | -Br | $-CH_2Cl$ | 1 | 5-Cl |
| 21 | -Cl | -H | -Cl | $-CH_2Cl$ | 1 | 6-Cl |
| 22 | -Br | -H | -Br | $-CH_2Cl$ | 1 | 6-Cl |
| 23 | -Br | -H | -Br | $-CH_2Cl$ | 1 | 7-Cl |
| 24 | -Cl | -H | -Cl | $-CH_2Cl$ | 1 | 6-F |
| 25 | -Br | -H | -Br | $-CH_2Cl$ | 1 | 6-F |
| 26 | -Cl | -H | -Cl | $-CH_2Cl$ | 1 | $5-NO_2$ |

TABLE I (Continued)

| Ex. No. | A | B | X | Y | n | Z |
|---|---|---|---|---|---|---|
| 27 | -Br | -Br | -H | $-CH_2Cl$ | 1 | $5-NO_2$ |
| 28 | -Cl | -Cl | -H | $-CH_2Cl$ | 1 | 7-Cl |
| 29 | -Cl | -Cl | -H | $-CH_2Cl$ | 1 | $6-NO_2$ |
| 30A | -Br | -Br | -H | $-CH_2Cl$ | 1 | $6-NO_2$ |
| 30B | -Br | -Br | -H | $-CH_2Cl$ | 1 | $6-NO_2$ |
| 31 | -Br | -Br | -H | $-CH_2Cl$ | 1 | $6-NH_2$ |
| 32 | C-C bond | | -Cl | -H | 0 | - |
| 33 | C-C bond | | -Br | -H | 0 | - |
| 34 | C-C bond | | $CH_2Cl$ | -H | 0 | - |
| 35 | C-C bond | | -H | -Cl | 0 | - |
| 36 | C-C bond | | -H | $-CH_2Br$ | 0 | - |
| 37 | C-C bond | | -H | $-CH_2Cl$ | 1 | 6-Cl |
| 38 | C-C bond | | -H | $-CH_2Cl$ | 1 | 6-F |

TABLE IA

| Example No. | M.pt. °C | Analysis C Calc | C Found | H Calc | H Found | N Calc | N Found |
|---|---|---|---|---|---|---|---|
| 5 | 131-132 | 28.7 | 28.5 | 1.9 | 1.8 | 3.4 | 3.2 |
| 6 | 115-116 | 31.3 | 31.2 | 2.3 | 2.5 | 3.7 | 4.0 |
| 7 | 128-132 | 38.5 | 39.9 | 2.9 | 3.1 | 4.1 | 4.5 |
| 8 | 123-125 | 30.4 | 31.7 | 2.3 | 2.5 | 3.2 | 3.7 |
| 9 | 132-133 | 27.6 | 28.4 | 2.1 | 2.4 | 2.9 | 3.4 |
| 10 | | 45.9 | 45.9 | 3.5 | 3.1 | 4.9 | 5.4 |
| 11 | 114-116 | 27.7 | 28.3 | 2.1 | 2.0 | 2.9 | 2.9 |
| 12 | 131-132 | 32.3 | 33.5 | 2.9 | 3.1 | 2.5 | 2.4 |
| 13 | 93-94 | 45.4 | 45.3 | 4.0 | 4.2 | 3.5 | 3.9 |
| 14 | 97-100 | 33.2 | 33.0 | 2.8 | 2.7 | 3.5 | 3.3 |
| 15 | 122-124 | 34.3 | 34.2 | 2.9 | 2.9 | 3.1 | 3.4 |
| 16 | 129-132 | 41.8 | 41.5 | 2.8 | 2.9 | 3.1 | 3.4 |
| 17 | 154-156 | 33.6 | 33.7 | 2.6 | 2.7 | 12.1 | 11.8 |
| 18 | 220-230 (dec.) | 37.4 | 37.8 | 2.4 | 2.5 | 4.8 | 4.6 |
| 19 | 35-36 | 35.0 | 34.2 | 2.4 | 2.4 | 3.7 | 3.5 |
| 20 | 100-101 | 28.4 | 28.4 | 1.9 | 2.3 | 3.0 | 3.0 |
| 21 | | 35.0 | 34.3 | 2.4 | 2.6 | 3.7 | 3.7 |
| 22 | 151-152 | 28.4 | 29.1 | 1.9 | 2.3 | 3.0 | 3.3 |
| 23 | | 28.4 | 28.7 | 1.9 | 2.3 | 3.0 | 3.3 |
| 24 | | 36.0 | 36.0 | 2.5 | 2.8 | 3.9 | 4.3 |
| 25 | 125 | 29.4 | 29.3 | 2.0 | 2.1 | 3.1 | 2.9 |
| 26 | 47-48 | 34.1 | 34.1 | 2.3 | 2.8 | 7.2 | 7.2 |
| 27 | 121-122 | 27.7 | 28.1 | 1.9 | 2.2 | 5.9 | 5.8 |
| 28 | 62.5 | 35.0 | 34.6 | 2.4 | 2.6 | 3.7 | 3.7 |
| 29 | 153 (dec.) | 34.1 | 34.2 | 2.3 | 2.7 | 7.2 | 6.7 |

TABLE IA (Continued)

| Example No. | M.pt. °C | Analysis C Calc | C Found | H Calc | H Found | N Calc | N Found |
|---|---|---|---|---|---|---|---|
| 30A | 212 (dec.) | 27.7 | 28.6 | 1.9 | 2.1 | 5.9 | 5.8 |
| 30B | 169.5 | 27.7 | 27.9 | 1.9 | 2.1 | 5.9 | 6.1 |
| 31 | 190 (dec.) | 29.6 | 30.4 | 2.5 | 2.8 | 6.3 | 6.4 |
| 32 | 127-128 | 46.6 | 46.5 | 3.1 | 3.1 | 5.4 | 5.6 |
| 33 | 142-143 | 39.7 | 40.0 | 2.6 | 2.8 | 4.6 | 5.0 |
| 34 | 80-81 | 48.6 | 48.7 | 3.7 | 3.9 | | |
| 35 | 74-76 | 46.6 | 46.5 | 3.1 | 3.2 | 5.4 | 5.3 |
| 36 | 125-126 | 41.8 | 43.0 | 3.2 | 3.2 | 4.4 | 4.6 |
| 37 | 88 | 43.2 | 43.7 | 3.0 | 3.1 | 4.6 | 4.9 |
| 38 | 82 | 45.6 | 45.7 | 3.1 | 3.3 | 4.8 | 4.8 |

Example 39

The fungicidal activity of compounds of the invention was investigated by means of the following tests.

(a) Antisporulant activity against vine downy mildew (Plasmopara viticola; Pva)

The test is a direct antisporulant one using a foliar spray. The lower surfaces of leaves of whole vine plants (cv Cabernet Sauvignon) are inoculated by spraying with an aqueous suspension containing $10^4$ zoosporangia/ml 2 days prior to treatment with the test compound. The inoculated plants are kept for 24 hours in a high humidity compartment, then 24 hours at glasshouse ambient temperature and humidity. Infected leaves are sprayed on their lower surfaces with a solution of active material in 1:1 water/acetone containing 0.04% "TWEEN 20" (Trade Mark; a polyoxyethylene sorbitan ester surfactant). The spraying is carried out with a moving track sprayer giving an application rate of 1kg/ha. After spraying, the plants are returned to normal glasshouse conditions for 96 hours and are then transferred to the high humidity compartment for 24 hours to induce sporulation, prior to assessment. Assessment is based on the percentage of the leaf area covered by sporulation compared with that on control leaves.

(b) Direct protectant activity against vine downy mildew (Plasmopara viticola; Pvp)

The test is a direct protectant one using a foliar spray. The lower surfaces of leaves of whole vine plants (cv Cabernet Sauvignon) are sprayed with the test compound at a dosage of 1 kilogram of active material per hectare using a track sprayer as described under (a), and after a subsequent 24 hours under normal glasshouse conditions the lower surfaces of the leaves are inoculated by spraying with an aqueous solution containing $10^4$ zoosporangia/ml. The inoculated plants are kept for 24 hours in a high humidity compartment, 5 days under normal glasshouse conditions and then returned for a further 24 hours to high humidity. Assessment is based on the percentage of leaf area covered by sporulation compared with that on control leaves.

(c) Direct protectant activity against vine grey mould (Botrytis cinerea; Bcp)

The test is a direct protectant one using a foliar spray. The lower surfaces of detached vine leaves (cv Cabernet Sauvignon) are sprayed with the test compound at a dosage of 1kg/ha using a track sprayer as in

(a). 24 hours after spraying the leaves are inoculated with droplets of aqueous suspension containing $10^5$ conidia/ml. After a further 5 days in high humidity the preçentage of leaf area covered by disease is assessed.

(d) Activity against apple powdery mildew (Podosphaera leucotricha; Pl)

The test is a direct therapeutic one using a foliar spray. The upper surfaces of apple seedlings are inoculated by spraying with an aqueous suspension containing $10^5$ conidia/ml 2 days prior to treatment with the test compounds. The inoculated plants are immediately dried and kept at glasshouse ambient temperatures and humidity prior to treatment. The plants are sprayed with the test compound at a dosage of 1 kilogram of active material per hectare using a track sprayer as described under (a). After drying the plants are returned to a compartment at 20-25° C and moderate humidity for up to 9 days, followed by assessment. Assessment is based on the percentage of the leaf area covered by sporulation compared with that on leaves of control plants.

(e) Activity against wheat leafspot (Leptosphaeria nodorum; Ln.)

The test is a direct therapeutic one, using a foliar spray. Leaves of wheat plants (cv Mardler), at the single leaf stage, are inoculated by spraying with an aqueous suspension containing 1 x $10^6$ spores/ml. The inoculated plants are kept for 24 hours in a high humidity compartment prior to treatment. The plants are sprayed with a solution of the test compound at a dosage of 1 kilogram of active material per hectare using a track sprayer as described under (a). After drying, the plants are kept for 6-8 days at 20-25° C and moderate humidity, followed by assessment. Assessment is based on the density of lesions per leaf compared with that on leaves of control plants.

(f) Activity against barley powdery mildew (Erysiphe graminis f.sp. hordei; Eg)

The test is a direct therapeutic one, using a foliar spray. Leaves of barley seedlings, (cv. Golden Promise) are inoculated by dusting with mildew conidia one day prior to treatment with the test compound. The inoculated plants are kept overnight at glasshouse ambient temperature and humidity prior to treatment. The plants are sprayed with the test compound at a dosage of 1 kilogram of active material per hectare using a track sprayer as described under (a). After drying, plants are returned to a compartment at 20-25° C and moderate humidity for up to 7 days, followed by assessment. Assessment is based on the percentage of leaf area covered by sporulation compared with that on leaves of control plants.

(g) Activity against wheat brown rust (Puccinia recondita; Pr)

The test is a direct protectant one using a foliar spray. Wheat seedlings (cv Brigand) are grown to the 1-1 $\frac{1}{2}$ leaf stage. The plants are then sprayed with the test compound at a dosage of 1 kg/ha using a track sprayer as described under (a). Test compounds are applied as solutions or suspensions in a mixture of acetone and water (50:50 v/v) containing 0.4% surfactant ("TWEEN 20" - Trade Mark).

18-24 hours after treatment, the seedlings are inoculated by spraying the plants from all sides with an aqueous spore suspension containing about $10^5$ spores/ml. For 18 hours after inoculation, the plants are kept in high humidity conditions at a temperature of 20-22° C. Thereafter, the plants are kept in ambient glasshouse conditions, that is, in moderate relative humidity and at a temperature of 20° C.

The disease is assessed 10 days after inoculation on the basis of the percentage of the plant covered by sporulating pustules compared with that on the control plants.

(h) Activity against rice leaf blast (Pyricularia oryzae; Po)

The test is a direct therapeutic one using a foliar spray. The leaves of rice seedlings (about 30 seedlings per pot) are sprayed with an aqueous suspension containing $10^5$ spores/ml 20-24 hours prior to treatment with the test compound. The inoculated plants are kept overnight in high humidity and then allowed to dry before spraying with the test compound at a dosage of 1 kilogram of active material per hectare using a track sprayer as described under (a). After treatment the plants are kept in a rice compartment at 25-30° C and high humidity. Assessments are made 4-5 days after treatment and are based on the density of necrotic lesions per leaf when compared with control plants.

(i) Activity against tomato early blight (Alternaria solani; As)

This test measures the contact prophylactic activity of test compounds applied as a foliar spray.

Tomato seedlings (cv Outdoor Girl) are grown to the stage at which the second true leaf is expanded. The plants are treated using a track sprayer as described under (a). Test compounds are applied as solutions or suspensions in a mixture of acetone and water (50:50 v/v) containing 0.04% surfactant ("TWEEN 20" - Trade Mark).

One day after treatment the seedlings are inoculated by spraying the leaf upper surfaces with a suspension of A. solani conidia containing $10^4$ spores/ml. For 3 days after inoculation plants are kept moist in a glasshouse compartment at or near 100% RH and 21° C. Thereafter plants are kept under humid, but not saturated, conditions. Disease is assessed 7 days after inoculation, based on the density and spread of lesions.

(j) Activity against wheat eyespot in-vitro (Pseudocercosporella herpotrichoides; Phl)

This test measures the in vitro activity of compounds against the fungus causing wheat eyespot.

The test compound is dissolved or suspended in acetone and is added to molten half strength Potato Dextrose Agar to give a final concentration of 100ppm compound and 3.5% acetone. After the agar has set, plates are inoculated with 6mm diameter plugs of agar/mycelium taken from a 14 day old culture of P. herpotrichoides.

Plates are incubated at 20° C for 12 days and radial growth from the inoculation plug is measured.

(k) Activity against Fusarium in-vitro (Fusarium species; Fsl)

This test measures the in vitro activity of compounds against a species of Fusarium that causes stem and root rots.

Compound is dissolved or suspended in acetone and added to molten half strength Potato Dextrose Agar to give a final concentration of 100ppm compound and 3.5% acetone. After the agar has set, plates are inoculated with 6mm diameter plugs of agar and mycelium taken from a 7 day old culture of Fusarium sp..

Plates are incubated at 20° C for 5 days and radial growth from the plug is measured.

The extent of disease control in all the above tests is expressed as a rating compared with either an untreated control or a diluent-sprayed-control, according to the criteria:-

0 = less than 50% disease control
1 = about 50-80% disease control
2 = greater than 80% disease control

The results of these tests are set out in Table II below:-

TABLE II

| Compound Example No. | Fungicidal Activity Pva | Pvp | Bcp | Pl | Ln | Eg | Pr | Po | As | PhI | FsI |
|---|---|---|---|---|---|---|---|---|---|---|---|
| 1 | | 1 | | | | | | | | | |
| 2 | | 1 | | 2 | | 2 | | | | 1 | |
| 3 | 1 | 1 | | | | 1 | | | | 1 | 1 |
| 4 | | 2 | | | | 2 | | | | 1 | |
| 5 | | | | 2 | | | | | | | |
| 6 | | 1 | | | | 2 | | | | 1 | |
| 7 | | | | | | 2 | | | | | |
| 8 | | 1 | | | | | 1 | | 1 | 1 | 1 |
| 9 | | 2 | | | | | | | 1 | 1 | 1 |
| 10 | | 2 | | | 1 | | 1 | | 2 | | 1 |
| 11 | | 2 | | | 1 | 2 | | | 1 | 1 | |
| 12 | | | | | | | | | 1 | | |
| 13 | | 2 | | | | | | | | | |
| 14 | | 2 | | | | 2 | | | 1 | 1 | 1 |
| 15 | | 1 | | | | | 1 | | | 1 | 1 |

TABLE II (Continued)

| Compound Example No. | Fungicidal Activity | | | | | | | | | | |
|---|---|---|---|---|---|---|---|---|---|---|---|
| | Pva | Pvp | Bcp | Pl | Ln | Eg | Pr | Po | As | PhI | FsI |
| 16 | | 2 | | | | | | | | | 1 |
| 17 | | 2 | | | | | | | | | 1 |
| 18 | | | | | | 2 | | | 2 | | |
| 19 | | 2 | | | | | | | | | |
| 20 | 1 | 1 | | | | 1 | | | | | |
| 22 | | | | | | | | | 1 | | |
| 23 | | 1 | | | | | | | | | |
| 26 | | 1 | | | | | 1 | | | | |
| 27 | | 2 | | | | 2 | | | | | |
| 28 | | 1 | | | | | | 1 | | | |
| 29 | | 1 | | | | | | | | | |
| 30A | | | | | | | 1 | | | | |
| 30B | | 1 | | | | | | | | | 1 |
| 31 | | 1 | 1 | | | | | | | | 1 |
| 32 | | | | | | | | | | | 1 |

TABLE II (Continued)

| Compound Example No. | Fungicidal Activity | | | | | | | | | | |
|---|---|---|---|---|---|---|---|---|---|---|---|
| | Pva | Pvp | Bcp | Pl | Ln | Eg | Pr | Po | As | PhI | FsI |
| 33 | | 1 | | | | | | | | 1 | 1 |
| 34 | 1 | 2 | | | | | | | | | 1 |
| 35 | | | | | | 1 | | | | | |
| 36 | 1 | | | | | | 1 | | | | |
| 37 | | | | | 1 | | | | | | |
| 38 | 1 | | | | | | | | | | |

Example 40

The fungicidal activity of certain compounds of the invention was further investigated by means of the following test.

Activity against barley powdery mildew (Erysiphe graminis f.sp. hordei; Eg)

The test is a direct therapeutic one, using a foliar spray. Leaves of barley seedlings (cv. Golden Promise) are inoculated by dusting with mildew conidia one day prior to treatment with the test compound. The inoculated plants are kept overnight at glasshouse ambient temperature and humidity prior to treatment. The plants are sprayed with a solution of the test compound in 1:1 water/acetone containing 0.04% "TWEEN 20" (Trade Mark: a polyoxyethylene sorbitan ester surfactant) at dosages of 0.01, 0.03 and 0.1 kilogram of active material per hectare using a moving track sprayer. Each treatment was replicated three times. After drying, plants are returned to a compartment at 20-25°C and moderate humidity for up to 7 days, followed by assessment. Assessment is based on the percentage of leaf area covered by sporulation compared with that on leaves of control plants.

Results

%D = per cent leaf area covered by disease
%E = per cent effect, relative to the controls.

| Dosage | Compound of Example 2A | | Compound of Example 2B | |
|---|---|---|---|---|
| Kg/ha | % D | % E | % D | % E |
| 0.01 | 73 | 0 | 2 | 97 |
| 0.03 | 29 | 60 | 0 | 100 |
| 0.1 | 3 | 96 | 0 | 100 |

Controls 73Diluents 71

**Claims**

1. A compound of the general formula

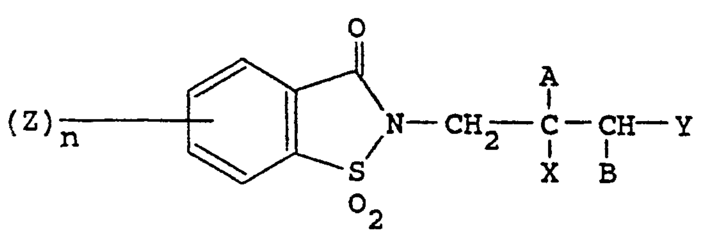

(I)

in which A and B independently represent a hydrogen or halogen atom or together represent a single carbon-carbon bond; X represents a hydrogen or halogen atom or an optionally substituted alkyl group or together with A represents an alkenyl group or together with B represents a -O- group; Y represents a hydrogen or halogen atom, a nitro, cyano, hydroxyl, formyl or carboxyl group or an optionally substituted alkyl, alkenyl, alkynyl, alkoxy, amino, alkylamino, dialkylamino, alkoxycarbonyl, alkanoyl, aroyl, alkylthio, alkylsulphinyl, alkylsulphonyl, carbamoyl, alkylamido, aryl, aralkyl or heterocyclyl group; each Z represents a halogen atom, nitro, cyano, hydroxyl, alkyl, haloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, carbamoyl or alkylamido group; and n represents an integer from 0 to 4; with the proviso that when A and X are both hydrogen atoms, neither B nor Y represents a hydrogen atom.

2. A compound according to claim 1 in which X represents a hydrogen or halogen atom or a $C_{1-6}$ haloalkyl group or together with A represents a $C_{1-4}$ alkenyl group or together with B represents a -O- group.

3. A compound according to claim 1 or claim 2 in which Y represents a hydrogen or halogen atom, a $C_{1-6}$ alkyl group, a $C_{1-6}$ alkoxycarbonyl group, an aryl group or a heterocyclyl-$C_{1-6}$ alkyl group, each group being optionally substituted by one or more substituents selected from halogen atoms, nitro, cyano,

hydroxyl, $C_{1-4}$ haloalkyl, $C_{1-4}$ alkoxy, amino, $C_{1-4}$ alkylamino, di-$C_{1-4}$alkylamino, formyl, $C_{1-4}$ alkoxycarbonyl and carboxyl groups.

4. A compound according to any one of claims 1,2, and 3 in which n is O or 1.

5. A compound according to any preceding claim in which A represents a hydrogen, chlorine or bromine atom, B represents a hydrogen, chlorine, bromine or iodine atom or together with A represents a single carbon-carbon bond, X represents a hydrogen, chlorine or bromine atom or a chloromethyl or bromomethyl group or together with A represents a $=CH_2$ group or together with B represents a -O- group; Y represents a hydrogen, chlorine or bromine atom or a methyl, chloromethyl, bromomethyl, tri(chloromethyl)methyl, ethoxycarbonyl, phenyl, triazolylmethyl or 1,1,3-trioxo-benzisothiazol-2-ylmethyl group; and n is O.

6. A compound according to any one of claims 1 to 4 in which A and X independently represent a hydrogen, chlorine or bromine atom; B represents a chlorine or bromine atom or together with A represents a single carbon-carbon bond; Y represents a chloromethyl group; n is 1; and Z represents a fluorine or chlorine atom or a nitro or amino group.

7. A compound according to any one of claims 1 to 4 in which A represents a hydrogen or halogen atom; B represents a halogen atom; X represents a hydrogen or halogen atom or an optionally substituted alkyl group; Y represents a halogen atom, a nitro, cyano, hydroxyl, formyl or carboxyl group or an optionally substituted alkyl, alkenyl, alkynyl, alkoxy, amino, alkylamino, dialkylamino, alkoxycarbonyl, alkanoyl, aroyl, alkylthio, alkylsulphinyl, alkylsulphonyl, carbamoyl, alkylamido, aryl, aralkyl or heterocyclyl group; each Z represents a halogen atom, nitro, cyano, hydroxyl, alkyl, haloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, carbamoyl or alkylamido group; and n represents an integer from 0 to 4; with the further provisos that A is not the same as X and B is not the same as Y; in the form of isomers having the same stereochemical configuration as that enantiomeric pair of isomers of N-(4-chloro-2,3-dibromobutyl)saccharin which has a melting point of 143° C.

8. A process for the preparation of a compound of formula I as defined in any one of claims 1 to 7 which comprises

(a) reacting a compound of the general formula

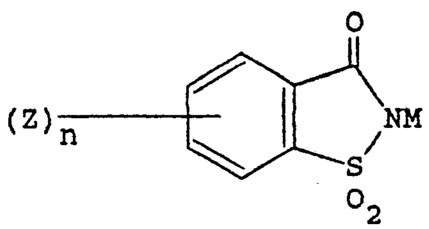

(II)

in which Z and n are as defined in any preceding claim and M represents a hydrogen or metal atom or an $NH_4$ group, with a compound of the general formula

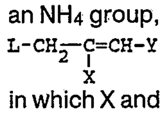

(III)

in which X and Y are as defined in any preceding claim and L represents a leaving group;

(b) if desired, reacting the compound obtained in (a) with a compound of the general formula

A - B (IV)

in which A and B independently represent a hydrogen or halogen atom; and

(c) if desired, subjecting a compound obtained in (b), in which A, B, X, Y, n and Z are as defined in claim 7, to isomer separation.

9. A process according to claim 8 in which the isomer separation is accomplished by

(i) treating a racemic mixture of four isomers of the compound with a solvent which selectively dissolves a first enantiomeric pair of isomers, filtering off a second enantiomeric pair of isomers and then isolating the first enantiomeric pair of isomers; or

(ii) subjecting a racemic mixture of four isomers of the compound to chromatography and isolating the desired isomers.

10. A fungicidal composition which comprises a carrier and, as active ingredient, a compound of formula I as defined in any one of claims 1 to 7.

11. A composition according to claim 10 which comprises at least two carriers, at least one of which is a surface-active agent.

12. A method for combating fungus at a locus which comprises treating the locus with a compound of formula I as defined in any one of claims 1 to 7 or with a composition as defined in claim 10 or claim 11.

13. A method according to claim 12 in which the locus comprises plants subject to or subjected to fungal attack, seeds of such plants or the medium in which the plants are growing or are to be grown.

14. The use as a fungicide of a compound of formula I as defined in any one of claims 1 to 7 or a composition as defined in claim 10 or claim 11.

European Patent Office

# EUROPEAN SEARCH REPORT

Application Number

EP 89 20 0009

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 80, no. 13, 1st April 1974, page 78, abstract no. 67415j, Columbus, Ohio, US; & JP-A-73 05 906 (KUMIAI CHEMICAL INDUSTRY CO., LTD) 21-02-1973<br>* Abstract * | 1,2,4-6,10-14 | C 07 D 275/06<br>C 07 D 417/06<br>A 01 N 43/80 |
| A | WO-A-8 607 590 (FMC CORP.)<br>* Claims; pages 39-41 * | 1,10-14 | |
| A | EP-A-0 110 219 (BASF)<br>* Claims * | 1 | |
| A | CHEMICAL ABSTRACTS, vol. 95, no. 9, 31st August 1981, page 787, abstract no. 80939n, Columbus, Ohio, US; & JP-A-81 18 971 (MITSUI TOATSU CHEMICALS, INC.) 23-02-1981<br>* Abstract * | 1 | |
| | | | TECHNICAL FIELDS SEARCHED (Int. Cl.4) |
| | | | C 07 D 275/00<br>C 07 D 417/00 |

The present search report has been drawn up for all claims

| Place of search | Date of completion of the search | Examiner |
|---|---|---|
| THE HAGUE | 22-03-1989 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document